# EUROPEAN PATENT SPECIFICATION

(11) **EP 3 068 375 B1**
(45) Date of publication and mention of the grant of the patent: **22.04.2020**
(21) Application number: 14859665.3
(22) Date of filing: 09.11.2014
(51) Int. Cl.: A61K 9/08, A61K 31/185, A61K 31/352, A61K 31/122, A61P 37/08

(54) **FORMULATIONS AND METHODS FOR PREVENTION AND TREATMENT OF ORAL ALLERGY SYNDROME**
FORMULIERUNGEN UND VERFAHREN ZUR PRÄVENTION UND BEHANDLUNG VON ORALEM ALLERGIESYNDROM
FORMULATIONS ET MÉTHODES DE PRÉVENTION ET DE TRAITEMENT DU SYNDROME D'ALLERGIE ORALE

(30) Priority: 11.11.2013 US 201314076263
(43) Date of publication of application: 21.09.2016
(73) Proprietor: Harish, Ziv, Tenaly, NJ 07670 (US)
(72) Inventor: Harish, Ziv, Tenaly, NJ 07670 (US)
(74) Representative: Avidity IP
(86) International application number: PCT/US2014/064713
(87) International publication number: WO 2015/070116

(56) References cited:
- WO-A2-2012/092594
- WO-A2-2013/116798
- US-A- 2 966 284
- US-A1- 2003 095 925
- US-A1- 2004 259 952
- US-A1- 2007 113 920
- US-A1- 2013 017 247
- US-A1- 2014 370 086

## Description

### Cross-references to Related Applications

This application is a continuation-in-part (CIP) of U.S. Application Serial Number 13/352,277 (REIZ-002DIV) filed January 17, 2012 and entitled " TREATMENT AND/OR PREVENTION OF ORAL ALLERGIC SYMPTOMS CAUSED BY ORAL CONTACT WITH FRUITS AND/OR VEGETABLES" which is a divisional application of U.S. Application Serial No. 12/981,726 (REIZ-002 - abandoned), filed December 30, 2010 and entitled "PREPARATIONS FOR TOPICAL PREVENTION AND/OR TREATMENT OF ORAL ALLERGIC SYMPTOMS DUE TO ORAL CONTACT WITH FRUITS AND/OR VEGETABLES".

### Field of the Invention

This invention relates generally to Oral Allergy Syndrome, and particularly to the prevention and treatment thereof.

### Background of the Invention

Oral Allergy Syndrome (OAS), also referred to as Pollen-associated Food Allergy Syndrome (PFAS), is a combination of symptoms that include oral itchiness, tingling and swelling of the lips, tongue and throat, as well as hoarseness and laryngeal tightness. About 1-2% of patients experience more severe reactions. OAS is experienced by individuals who are allergic to pollen when they ingest raw fruits and vegetables. It is a localized IgE-mediated allergic reaction that is triggered by the contact of cross-reacting raw fruits and vegetables with the oral mucosa and/or pharyngeal mucosa of pollen-sensitized individuals. This allergic reaction occurs when sensitized mast cells in the oral/ pharyngeal mucosa that are coated by pollen-specific IgE antibodies come in contact with raw fruits or vegetables containing proteins that cross-react with those of specific pollen proteins. This contact triggers the sensitized mast cells to release their mediators such as histamine. These mediators exert their effect on target tissues having blood vessels, and causes the blood vessels to dilate and become permeable, resulting in tissue swelling. These mediators also stimulate neural receptors, which results in itchiness.

Individuals sensitive to birch-tree pollen often react to fruits of the Rosacae family such as fresh or raw apples, pears, quinces, peaches, strawberries, rasberries, cherries, plums, apricots, and peaches as well as to carrots and almonds. Individuals who are allergic to ragweed react to melons and those allergic to grass and weed pollen often experience OAS symptoms with cucumber, celery, green pepper, avocado and banana. Many individuals are allergic to multiple pollen groups, and thus they experience symptoms with the ingestion of all of the above fruits and vegetables.

With increasing epidemiologic and clinical research, diagnostic criteria have been revised on multiple occasions over the past 20 years. Estimates of the percentage of patients with pollen allergy who also suffer from OAS vary from 47% to 70%, and this is thought to be the most common food allergy in adolescents and adults. Similarly other authors noticed that Pollen-associated Food Allergy syndrome represents the most common food allergy in adults with 23-76% of patients with allergic rhinitis to pollen showing sensitivity to one or more foods.

The National Institute of Allergy and Infectious Diseases, or NIAID, estimate the prevalence of pollen allergy at about 12.87 percent. That is roughly equivalent to 1 in 7 individuals affected with this hypersensitivity in the USA: 35 million individuals, while the review of Mayo Clinic noticed that 27.2 million people in USA suffer from allergic rhinitis/hay fever (Appendix I). In Western Europe, the prevalence of clinically confirmable allergic rhinitis was estimated to be 23% while in Japan, the prevalence of allergic rhinitis was estimated to be 39.4% and that of pollinosis was 29.8%.

Based on such data, and being said that OAS afflicts approximately 50 percent of the population suffering from pollinosis and allergic rhinitis, the targeted population will be about 13.6-17.5 million Americans suffering from OAS. Conservative extrapolations suggest that about 15.0 million, 12.0 million and 7.2 million people suffer from OAS in Europe (15 countries), in Japan and in South Korea, respectively.

Currently, there is no treatment approved for OAS. Individuals afflicted with this condition often dejectedly avoid all raw fruits and vegetables; some individuals with milder symptoms continue having the fruits and vegetables, despite significant discomfort associated with the ingestion thereof. Anti-histamines have not been found to be effective for this condition. In fact, taking anti-histamines daily for hay-fever symptoms (pollinosis) does not prevent individuals who take them from experiencing OAS symptoms.

Patent application US-20140370086 published on 27-04-2016, with priority date 17-06-2013 teaches the same formulation for use in treatment of oral allergy syndrome, proposing Cromolyn sodium solution containing Cromolyn 100-200 mg per 5 ml of sterile water or glycerine but without a taste masking agent present.

The patents WO-2012092594 and WO-2013/116798 teach same formulation for use in treatment of oral allergy syndrome using 100 mg of Cromolyn Sodium/ 5cc water (e.g.Gastrocrom™), but not at higher concentrations.

The patent US 2003/095925 teaches a buccal spray composition for transmucosal administration of a mast cell mediator like cromolyn as salt, but not used for treating oral food allergies.

### Summary of the Invention

The scope of this invention is defined by the claims. Any references in the description to methods of treatment refer to the compounds, pharmaceutical compositions and medicaments of the present invention for use in a method for treatment of the human (or animal) body by therapy.

OAS (PFAS) is the most common food allergy estimated to occur in 47-70% of pollen allergic individuals, and in roughly 10% of the population. Currently, there is no approved treatment for OAS, except to recommend avoidance of the fruits or vegetables that provoke the symptoms. An aqueous solution of Cromolyn sodium according to the invention applied to the lips and/or to the oral cavity prior to contact with the food allergen appears in the Scout trial to successfully prevent and treat symptoms of OAS in sensitive individuals.

Cromolyn is one of the most effective, non-sedating anti-allergic drugs, with an exceptional safety profile, which has been used for allergic condition for decades. The series of successive pilot studies and proof-of-concept studies have provided supporting clinical results. The safe history of Cromolyn and the startlingly effective clinical experience gained thus far over the most recent two years allow the confident prediction of safe and effective future outcomes.

According to the invention, Cromolyn sodium has demonstrated an ability to prevent and treat the symptoms related to OAS. The strength of the safety track record of Cromolyn sodium is reinforced by the clinical safety database collected so far.

According to the invention, new formulations, uses, methods, and protocols have been identified for Cromolyn Sodium for the indication of preventing and/or treating Oral Allergy Syndrome (OAS), also referred to as Pollen-associated Food Allergy Syndrome (PFAS).

According to the invention , Cromolyn substantially adheres to the oral mucosa, and in our studies exerted substantially immediate effects in aqueous solution. Therefore, mucoadheseive or glycerin is NOT needed.

Pilot and Phase IIa Proof of Concept studies have been performed with complete results already available, so the formulations are ready to enter the Phase IIb stage of clinical development in the indication of Prevention and/or Treatment of Oral Allergy Syndrome"

The exciting clinical results using the formulations set forth herein will establish various aspects of the invention as the most efficient and safe treatment and prevention of Oral Allergy Syndrome (OAS) or Pollen-associated Food Allergy Syndrome (PFAS), a therapy that is not only strikingly efficacious, but also dramatically improves patient Quality of Life.

According to the invention claim 1 states a pharmaceutical formulation for use in the prevention and/ or treatment of oral allergy syndrome, the formulation comprising: a solution of Cromolyn sodium in water at a concentration of at least 150 mg Cromolyn sodium per 5 ml water, and
ataste masking agent, wherein the formulation is for topical application to the oral mucosa.

In some embodiments, the concentration is 200 mg Cromolyn sodium per 5 ml water.

In some embodiments, the concentration is 150 mg Cromolyn sodium per 5 ml water.

In some embodiments, the concentration is 250 mg Cromolyn sodium per 5 ml water.

In some embodiments, the formulation further includes flavoring.

In some embodiments, the formulation is provided as a dose of at least 5 ml.

In some embodiments, the formulation is provided in a spray bottle.

In some embodiments, the formulation is provided using a bottle with a measuring dropper for measuring out 1 ml doses.

In some embodiments, the formulation is provided in a bottle with a cap for measuring out 2.5 ml, 5.0 ml, and 7.5 ml doses.

Another general aspect is a method for the prevention and/or treatment of Oral Allergy Syndrome, where the method includes: taking into the mouth a quantity of an aqueous solution at a concentration of 150 mg Cromolyn sodium per 5 ml water to 250 mg Cromolyn sodium per 5 ml water; swishing the solution around in the mouth for about two to ten seconds; and expectorating the solution.

In some embodiments, the concentration of aqueous solution of Cromolyn sodium is 200 mg per 5 ml water.

In some embodiments, the quantity taken into the mouth is 5 ml.

In some embodiments, the quantity taken into the mouth is 7.5 ml.

In some embodiments, the quantity taken into the mouth is introduced into the mouth via a spray.

In some embodiments, the quantity taken into the mouth is introduced into the mouth via an ampoule.

In some embodiments, the quantity taken into the mouth is introduced via a dropper.

In some embodiments, swishing the solution around in the mouth is performed for about five seconds.

In some embodiments, the aqueous solution further includes flavoring to make oral rinsing more pleasant.

In some embodiments, the aqueous solution further includes a taste masker to make the sensation of the Cromolyn sodium less unpleasant.

### Detailed Description of the Invention

Preliminary study: a first open label study was performed by Dr. Ziv Harish, an Allergist Immunologist associated with Englewood Hospital. This study (Scout Study) was approved by the hospital IRB committee.

Patients were selected from registry of individuals with OAS. Four patients were challenged with a small slice of an apple to confirm OAS related symptoms. After resolution of the symptoms, patients gargled 100 mg Cromolyn per 5 ml of water (aqeous solution) prior to a second challenge with apple. Two of the four patients had no symptoms after a single dose of the Cromolyn oral rinse (100 mg Cromolyn in 5 ml water). One patient experienced only minimal symptoms after the second challenge, however tolerated the whole apple. The fourth patient experienced similar symptoms to those experienced with the first challenge, however she responded instantly to a second application of Cromolyn oral rinse at a concentration of 100 mg Cromolyn in 5 ml of water. All four patients enjoyed eating a whole apple (which they had not done in years).

Phase IIa Proof of Concept study in OAS is an extension of the previous study. In this study, a randomized, double blind, placebo-controlled, 2-way cross-over study was performed in the same institution. The primary objective was to assess the efficacy of Cromolyn sodium solution in reducing symptoms related to Oral Allergy Syndrome.

For each participant, a set of two vials of Cromolyn solution was prepared: one contained the aqueous solution of Cromolyn sodium, and the other contained a placebo (both tasted similarly). The first visit included a challenge (apple) to ascertain that the individual indeed suffers from OAS. Once the symptoms (triggered by the ingestion of the apple) subsided, the participant was administered his/her designated study solution that contained either placebo or the aqueous Cromolyn solution. On the second visit the participant gargled the second part of his/her study solution of either placebo or Cromolyn solution that he/she did not take on the first visit. The Primary outcome measure was to determine the percentage of patients with OAS symptoms (Oral Itchiness, Lip swelling, Tongue swelling, Ear itchiness, Itchy throat, Flush, Shortness of breath, Lip itchiness, Cough) following treatment with the Cromolyn compared to placebo. Six patients completed the double blind placebo control trial. Two patients were complete responders with the 100 mg Cromolyn per 5 ml water dose and had no symptoms when they ate the apple following administration of the Cromolyn solution. Three required a second application of the solution (at a concentration of 100 mg Cromolyn sodium in 5 ml water) to alleviate on going symptoms that they experienced after the challenge with the apple, despite attempted prophylaxis by first applying the solution at a concentration of 100 mg. of Cromolyn in 5 ml water. These individuals responded instantly to additional application of the Cromolyn solution. Two patients (one who got only a single application of the Cromolyn solution at 100 mg Cromolyn in 5 ml water, and one who required the second application of Cromolyn at that concentration) had residual lip pruritus, which was immediately alleviated by applying Cromolyn solution directly to the lip with a Q-tip (see Table 1).

**Table 1: Results DBPC: Cromolyn vs. placebo. Patients challenged with raw apple**

| Patient # | Symptoms at baseline | | PlaceboCromolyn (100 mg in 5 ml water, then 2nd dose of 100 mg in 5 ml water when required) |
|---|---|---|---|
| 1 M.L | OI; ITH;F | OI; LS; TS; EI | |
| | Minor LI | | |

| Resolved with additional Cromolyn swab | | | |
|---|---|---|---|
| 2 M.C | SOB | SOB | Mild EI (questionably due to touching eye) |
| 3 M.Ch | OI; LS; SOB ITH | OI; LS | No symptoms |
| 4 R.O | OI; EI; TS; ITH; LI; TS | OI; CGH; TS | OI resolved with lip swab and 2nd application |
| 5 N.B | OI; ITH; EI; HRS | OI;ITH; HRS; EI | OI; ITH; HRS; EI Resolved with 2nd application |
| 6 S.B | OI; LS; TS; EI; | SOB | OI; TS |

| | | | |
|---|---|---|---|
| Resolved with 2nd application OI: Oral Itchiness - LS: Lip Swelling - TS: Tongue Swelling - EI: Ear Itchiness - ITH: Itchy Throat - F Flush - SOB: Shortness Of Breath - LI: Lip Itchiness - CGH: Cough | | | |

Interpretation and dose-rationale for next Phase IIb study:
All ten patients included in the clinical program had an immediate and dramatic response to the oral/pharyngeal application of Cromolyn sodium solution at a concentration of 100 mg Cromolyn sodium per 5 ml water. Four patients required a second application of 100 mg of Cromolyn sodium per 5 ml water to be completely symptom free. Four patients experienced residual lip pruritus, which was completely alleviated with applying Cromolyn directly to the lip. We therefore believe that a higher concentration of Cromolyn is required, e.g., 200 mg of Cromolyn sodium per 5 ml water. Given that Cromolyn sodium is safe, and is not swallowed, the higher concentration of 200 mg /5 ml. solution should not pose any additional safety risk.

For lip symptoms, given that the oral rinse had no contact with the mucosal surface of the lips, the occurrence of lip symptoms is not surprising. Cromolyn can be applied to the lips with a swab or Q-tip, even after the onset of pruritus, to completely and immediately alleviate the symptoms. A specific patent application prepared and filed previously, Application Serial No. 12/026,972 (Abandoned) filed 2/17/11, and it's progeny, Application Serial No. 13/365,040 filed 2/2/12 teaches and claims applying Cromolyn to the lips via a Cromolyn lipstick or swab.

According to the invention, the administration of the medication approximately 10 minutes before the ingestion of the raw fruit is expected to be sufficient for it to exert its preventive effect on symptom development.

Risks related to the topical application of Cromolyn are unlikely to occur as it is a well-known safe drug, and further, in its topical use for OAS it does not need to be swallowed.

The regulatory path to approval by the FDA or EMEA is believed to be relatively simple, since the drug is well known and in clinical use for decades, it has an impeccable safety record, and in this case it does not require systemic exposure (i.e., it is not swallowed). The only regulatory hurdle is that currently there is no approved drug for the specific indication of OAS. This entails convincing the regulatory authorities of the seriousness of OAS, the deprivation of individuals afflicted from the nutritional benefits of raw fruits and vegetables, and the psychological stigma associated with food allergy, balanced against the effectiveness and safety of the drug. It seems that this hurdle will be relatively simple to overcome.

Cromolyn sodium is disodium 5,5'-[(2-hydroxytrimethylene)dioxy]bis[4-oxo-4H-1-benzopyran-2-carboxylate].

The molecular structure of Cromolyn sodium is:
International Name: Cromolyn sodium
Chemical Name: Disodium 5,5'-[(2-hydroxytrimethylene)dioxy]bis[4-oxo-4H-1-benzopyran-2-carboxylate].
Molecular Formula: C23H14Na2O11

Cromolyn sodium is a water-soluble, odorless, white, hydrated crystalline powder. It is tasteless at first, but leaves a slightly bitter aftertaste. Cromolyn sodium is totally ionized in physiologic pH, and thus it has negligible fat solubility.

Cromolyn sodium has been used as one of the most effective, non-sedating anti-allergic drugs since the 1970's. In vitro and in vivo animal studies have shown that Cromolyn sodium inhibits sensitized mast cell degranulation, which occurs after exposure to specific antigens. Cromolyn sodium acts by inhibiting the release of mediators from mast cells. Studies show that Cromolyn sodium indirectly blocks calcium ions from entering the mast cell, thereby preventing mediator release. Cromolyn sodium inhibits both the immediate and non-immediate bronchoconstrictive reactions to inhaled antigen. Cromolyn sodium also attenuates bronchospasm caused by exercise, toluene, diisocyanate, aspirin, cold air, sulfur dioxide, and environmental pollutants. Cromolyn sodium has no intrinsic bronchodilator or antihistamine activity.

Cromolyn sodium has been safely used in a variety of allergic conditions including asthma, for which until recently Cromolyn sodium inhaler was frequently prescribed (Intal®). In 2009, it was taken off the market due to required changes in the propellant molecule from CFC to HFA and the inability to reformulate the product to a CFC-free or HFA propellant formulation (9). It is available over-the-counter (OTC) for allergic rhinitis (Nasalcrom®). It is available by prescription for allergic conjunctivitis (Opticrom® and Crolom®) as well as for mastocytosis affecting the gastrointestinal tract (Gastrocrom®) and for eczema (Altoderm®). In view of the extremely low occurrence of side effects experienced with swallowing Cromolyn sodium in treatment of GI ailments, NDL expects the topical application of Cromolyn sodium, in the form of an oral rinse, for example, to be at least as safe, and likely more so.

When Cromolyn sodium is administered via a power-driven nebulizer, under the brand names: Intal® and Intal Inhaler®, Cromolyn sodium liquid is made into an aerosol that one breathes in through one's mouth, or through one's mouth and nose, into the lungs. However, one is also commonly advised: "Do not take this medicine as a solution by mouth". One is also advised "If you get a bitter or unpleasant taste in your mouth, gargle or rinse your mouth after you use this medicine." Patients are also advised: "To relieve dry mouth or throat irritation caused by Cromolyn inhalation, rinse your mouth with water, chew gum, or suck sugarless hard candy after each treatment".

Gastrocrom® Oral Concentrate is intended for "oral" use, i.e., to be swallowed and reach superficially situated mast cells along the gastrointestinal tract, or to be absorbed systemically via the gastrointestinal tract, just as pills, capsules, and tablets are to be swallowed and absorbed systemically. Each 5 mL ampule of Gastrocrom® contains 100 mg Cromolyn sodium, USP, in purified water.

The physicochemical properties of Cromolyn impede its absorption across epithelial lining, such as through the oral mucosa, and contribute to its poor absorption from the gastrointestinal tract. No more than 1% of a dose administered orally is absorbed by humans, the remainder being excreted in the feces. In a phase 1 study with 12 volunteers, very little absorption of the product was seen after administration of 500 mg of Cromolyn sodium by mouth. From 0.28 to 0.50% of the administered dose was recovered in the first 24 hours of urinary excretion in 3 subjects. The mean urinary excretion of an orally administered dose over 24 hours in the remaining 9 subjects was 0.45%.

It is commonly accepted that Cromolyn sodium is poorly absorbed topically through mucosal barriers, and is consequently not absorbed well into the oral mucosa. Further, it is also commonly believed that the physicochemical properties including the polarity of the Cromolyn sodium molecule may also interfere with its absorption and local activity.

Since Cromolyn sodium is poorly absorbed through the GI tract, it has not been considered as a candidate for treatment of systemic food allergies.

The oral solution of Cromolyn of the invention is expected to remain effective for 4-6 hours. In our studies, in about 40% of patients tested, 100 mg Cromolyn in 5 ml water provided only partial relief of OAS symptoms, and so an additional application of oral solution of 100 mg Cromolyn in 5 ml water was needed. Based on further testing, the optimal dose was found to be 200 mg Cromolyn in 5 ml water, which was effective for 100% of the patients tested. Although Cromolyn is one of the safest medications known, we recommend spitting out the Cromolyn solution after swishing it in the mouth for about 5 seconds, and do NOT recommend swallowing it.

A solution of 200 mg Cromolyn in 5 ml of water can be provided in single-dose ampules, or can be provided in a multi-dose bottle having a measuring dropper cap or a measuring cup cap for measuring out the 5 ml dose of 200 mg Cromolyn in 5 ml water. It can also be provided in a metered pump spray bottle which provides, for example, 0.75 ml of 200 mg Cromolyn in 5 ml water per spray.

200 mg in 5 ml, 7.5 ml (about half a tablespoon or ten 0.75 ml sprays) gargled or swished around in the mouth all at once for about five seconds - best mode for an adult. Five sprays can also work for an adult: for example, one spray to the back of the throat, followed by one spray to the top, bottom, and both sides of the mouth.

5 ml (one teaspoon) is the appropriate volume for a child. Five sprays of 200 mg/5 ml is effective.

As is well-known to those of skill in the art of formulating oral rinses and sprays, to make the taste of the oral solution more pleasant, flavoring (cherry, strawberry, mint, lemon/lime, for example, and/or taste maskers to mute the salty and/or bitter taste of Cromolyn can be added to improve patient experience and therefore patient compliance.

## Claims

1. A pharmaceutical formulation for use in the prevention and/or treatment of oral allergy syndrome, the formulation comprising:
a solution of Cromolyn sodium in water at a concentration of at least 150 mg Cromolyn sodium per 5 ml water, and
a taste masking agent,
wherein the formulation is for topical application to the oral mucosa.

2. The formulation for use according to claim 1, wherein the concentration is 200 mg Cromolyn sodium per 5 ml water.

3. The formulation for use according to claim 1, wherein the concentration is 150 mg Cromolyn sodium per 5 ml water.

4. The formulation for use according to claim 1, wherein the concentration is 250 mg Cromolyn sodium per 5 ml water.

5. The formulation for use according to claim 1, further comprising: flavoring.

6. The formulation for use according to claim 1, wherein the formulation is provided as a dose of at least 5 ml.

## Patentansprüche

1. Pharmazeutische Formulierung zur Verwendung bei der Prävention und/oder Behandlung von oralem Allergiesyndrom, wobei die Formulierung Folgendes umfasst:
eine Lösung aus Cromolyn-Natrium in Wasser bei einer Konzentration von mindestens 150 mg Cromolyn-Natrium pro 5 ml Wasser und
ein Geschmackerzeugungsmittel,
wobei die Formulierung zur topischen Anwendung an der Mundschleimhaut dient.

2. Formulierung zur Verwendung nach Anspruch 1, wobei die Konzentration 200 mg Cromolyn-Natrium pro 5 ml Wasser beträgt.

3. Formulierung zur Verwendung nach Anspruch 1, wobei die Konzentration 150 mg Cromolyn-Natrium pro 5 ml Wasser beträgt.

4. Formulierung zur Verwendung nach Anspruch 1, wobei die Konzentration 250 mg Cromolyn-Natrium pro 5 ml Wasser beträgt.

5. Formulierung zur Verwendung nach Anspruch 1, ferner umfassend: Aroma.

6. Formulierung zur Verwendung nach Anspruch 1, wobei die Formulierung als eine Dosis von mindestens 5 ml bereitgestellt ist.

## Revendications

1. Formulation pharmaceutique pour une utilisation dans la prévention et/ou le traitement d'un syndrome d'allergie orale, la formulation comprenant :
une solution de cromolyn sodique dans de l'eau à une concentration d'au moins 150 mg de cromolyn sodique pour 5 ml d'eau, et
un agent de masquage de goût,
la formulation étant destinée à une application topique sur la muqueuse buccale.

2. Formulation pour une utilisation selon la revendication 1, dans laquelle la concentration est de 200 mg de cromolyn sodique pour 5 ml d'eau.

3. Formulation pour une utilisation selon la revendication 1, dans laquelle la concentration est de 150 mg de cromolyn sodique pour 5 ml d'eau.

4. Formulation pour une utilisation selon la revendication 1, dans laquelle la concentration est de 250 mg de cromolyn sodique pour 5 ml d'eau.

5. Formulation pour une utilisation selon la revendication 1, comprenant en outre : un arôme.

6. Formulation pour une utilisation selon la revendication 1, la formulation étant fournie à une dose d'au moins 5 ml.
